# EUROPÄISCHE PATENTSCHRIFT

(11) **EP 0 428 860 B1**
(45) Veröffentlichungstag und Bekanntmachung des Hinweises auf die Patenterteilung: **06.07.1994**
(21) Anmeldenummer: 90119551.1
(22) Anmeldetag: 12.10.1990
(51) Int. Cl.: C07D 215/14, A61K 31/47

(54) **Substituierte (Chinolin-2-yl-methoxy)phenyl-carbonyl-harnstoffe**
Substituted (quinolin-2-yl-methoxy)phenyl-carbonyl-ureas
(Quinolin-2-yl-méthoxy)phényl-carbonyl-urées substituées

(30) Priorität: 25.10.1989 DE 3935491
(43) Veröffentlichungstag der Anmeldung: 29.05.1991
(73) Patentinhaber: BAYER AG, 51368 Leverkusen (DE)
(72) Erfinder: Raddatz, Siegfried, Dr., W-5000 Köln 80 (DE); Mohrs, Klaus, Dr., W-5600 Wuppertal 1 (DE); Fugmann, Burkhard, Dr., W-5603 Wülfrath (DE); Fruchtmann, Romanis, W-5000 Köln 1 (DE); Kohlsdorfer, Christian, Dr., W-5042 Erftstadt (DE); Müller-Peddinghaus, Reiner, Prof.Dr., W-5060 Bergisch-Gladbach 2 (DE); Theissen-Popp, Pia, Dr., W-5060 Bergisch-Gladbach 2 (DE)

(56) Entgegenhaltungen:
- GB-A- 2 202 223
- CHEMICAL ABSTRACTS, Band 97, 1982, Seite 715, Zusammenfassung Nr. 127519j, Columbus, Ohio, US

## Beschreibung

Die Erfindung betrifft substituierte (Chinolin-2-yl-methoxy)phenyl-carbonylharnstoffe, Verfahren zu ihrer Herstellung und ihre Verwendung in Arzneimitteln.

Es ist bekannt, daß N-(2-Chinolin-2-yl-methoxy)phenyl-N′-alkyl-N′-alkoxyharnstoffe eine herbizide Wirkung besitzen [vgl. JP 82 64-675; 7.10.80-JP-14 00 91]. Außerdem werden dort auch N,N′-Dimethyl-N′-[3-(2-Chinolyl-methoxy)]phenylharnstoffe beschrieben.

Die Erfindung betrifft nun substituierte (Chinolin-2-yl-methoxy)phenyl-carbonylharnstoffe der allgemeinen Formel (I) 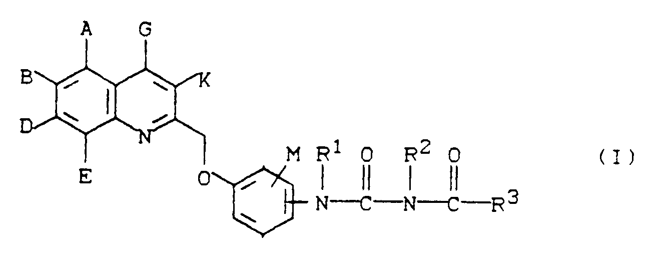
in welcher
- A, B, D, E, G, K und M: gleich oder verschieden sind und
- für Wasserstoff, Hydroxy, Halogen, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
- R¹ und R²: gleich oder verschieden sind und
- für Wasserstoff oder für geradkettiges oder verzweigtes Alkyl mit bis zu 10 Kohlenstoffatomen stehen, das gegebenenfalls durch Halogen, Aryl mit 6 bis 10 Kohlenstoffatomen oder durch Cycloalkyl mit 3 bis 8 Kohlenstoffatomen substituiert ist, oder
- für Cycloalkyl mit 3 bis 8 Kohlenstoffatomen stehen, oder
- für Aryl mit 6 bis 10 Kohlenstoffatomen stehen, das gegebenenfalls durch Halogen, Nitro, Cyano oder Trifluormethyl substituiert ist,
und
- R³: - für Aryl mit 6 bis 10 Kohlenstoffatomen steht, das gegebenenfalls bis zu 3-fach gleich oder verschieden durch Halogen, Nitro, Cyano, Trifluormethyl oder durch geradkettiges oder verzweigtes Alkyl mit bis zu 8 Kohlenstoffatomen substituiert ist, oder
- für geradkettiges oder verzweigtes Alkyl mit bis zu 10 Kohlenstoffatomen steht, das gegebenenfalls durch Cycloalkyl mit 3 bis 8 Kohlenstoffatomen, Phenyl, Carboxy, Halogen, Hydroxy oder durch Alkoxy oder Alkoxycarbonyl mit jeweils bis zu 8 Kohlenstoffatomen substituiert ist, oder
- für Cycloalkyl mit 3 bis 8 Kohlenstoffatomen steht,
und deren Salze.

Im Rahmen der vorliegenden Erfindung werden physiologisch unbedenkliche Salze bevorzugt. Physiologisch unbedenkliche Salze der substituierten (Chinolin-2-yl-methoxy)phenyl-carbonylharnstoffe können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugt sind Verbindungen der allgemeinen Formel (I),
in welcher
- A, B, D, E, G, K und M: gleich oder verschieden sind und
- für Wasserstoff, Hydroxy, Fluor, Chlor, Brom, Carboxy, Nitro, Trifluormethyl, Trifluormethoxy oder für geradkettiges oder verzweigtes Alkyl oder Alkoxy mit jeweils bis zu 6 Kohlenstoffatomen stehen, oder
- für Phenyl stehen, das gegebenenfalls durch Fluor, Chlor, Brom, Hydroxy, Nitro oder Cyano substituiert ist,
- R¹ und R²: gleich oder verschieden sind und
- für Wasserstoff oder geradkettiges oder verzweigtes Alkyl mit bis zu 8 Kohlenstoffatomen stehen, das gegebenenfalls durch Fluor, Chlor, Brom, Phenyl, Cyclopropyl, Cyclopentyl oder Cyclohexyl substituiert ist, oder
- für Cyclopropyl, Cyclopentyl oder Cyclohexyl stehen, oder
- für Phenyl stehen, das gegebenenfalls durch Fluor, Chlor, Brom, Nitro, Cyano oder Trifluormethyl substituiert ist,
und
- R³: - für Phenyl steht, das gegebenenfalls bis zu 2-fach gleich oder verschieden durch Fluor, Chlor, Brom, Nitro, Cyano, Trifluormethyl oder durch geradkettiges oder verzweigtes Alkyl mit bis zu 6 Kohlenstoffatomen substituiert ist, oder
- für geradkettiges oder verzweigtes Alkyl mit bis zu 8 Kohlenstoffatomen steht, das gegebenenfalls durch Cyclopropyl, Cyclopentyl oder Cyclohexyl, Fluor, Chlor, Brom, Hydroxy oder durch Alkoxy oder Alkoxycarbonyl mit jeweils bis zu 6 Kohlenstoffatomen substituiert ist, oder
- für Cyclopropyl, Cyclopentyl oder Cyclohexyl steht
und deren Salze.

Besonders bevorzugt sind Verbindungen der allgemeinen Formel (I), in welcher
- A, B, D, E, G, K und M: gleich oder verschieden sind und
- für Wasserstoff, Hydroxy, Fluor, Chlor, Brom oder geradkettiges oder verzweigtes Alkyl mit bis zu 4 Kohlenstoffatomen stehen,
- R¹ und R²: gleich oder verschieden sind und
- für Wasserstoff oder geradkettiges oder verzweigtes Alkyl mit bis zu 6 Kohlenstoffatomen stehen, das gegebenenfalls durch Phenyl oder Cyclohexyl substituiert ist, oder
- für Cyclopentyl oder Cyclohexyl stehen, oder
- für Phenyl stehen, das gegebenenfalls durch Fluor, Chlor oder Brom substituiert ist,
und
- R³: - für Phenyl steht, das gegebenenfalls bis zu 2-fach gleich oder verschieden durch Fluor, Chlor, Brom, Nitro oder Cyano substituiert ist, oder
- für geradkettiges oder verzweigtes Alkyl mit bis zu 6 Kohlenstoffatomen steht, das gegebenenfalls durch Cyclopropyl, Cyclopentyl oder Cyclohexyl substituiert ist, oder
- für Cyclopropyl, Cyclopentyl oder Cyclohexyl steht
und deren Salze.

Ganz besonders bevorzugt sind Verbindungen der allgemeinen Formel (I), in denen die Chinolylmethoxygruppierung am Phenyl in 4-Stellung zur Carbonylharnstoffgruppe steht.

Außerdem wurde ein Verfahren zur Herstellung der Verbindungen der allgemeinen Formel (I) 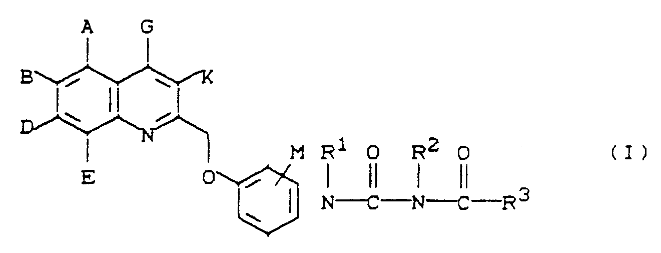
in welcher
A, B, D, E, G, K, M, R¹, R² und R³ die oben angegebene Bedeutung haben,
gefunden, das dadurch gekennzeichnet ist, das man Chinolylmethoxy-aniline der allgemeinen Formel (II) 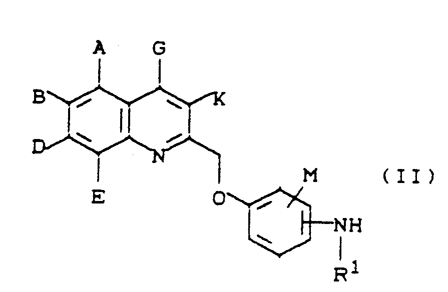
in welcher
A, B, D, E, G, K, M und R¹ die oben angegebene Bedeutung haben,
mit Isocyanaten der allgemeinen Formel (III)

R³-CO-NCO (III)

in welcher
R³ die oben angegebene Bedeutung hat,
in inerten Lösemitteln zu Verbindungen der allgemeinen Formel (Ia) 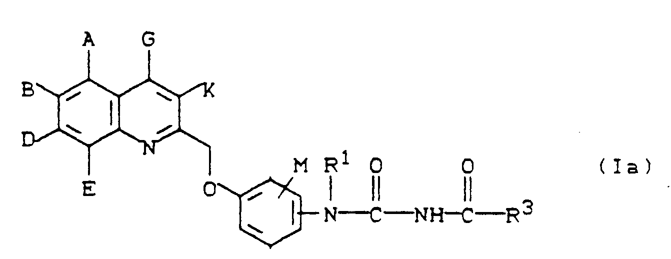
in welcher
A, B, D, E, G, K, M, R¹ und R³ die oben angegebene Bedeutung haben,
umsetzt und im Fall der Herstellung der Verbindungen der allgemeinen Formel (I) mit R² ≠ H anschließend die Verbindungen der Formel (Ia) mit Alkylierungsmitteln nach üblicher Methode in inerten Lösemitteln alkyliert.

Das Verfahren kann durch folgendes Formelschema erläutert werden: 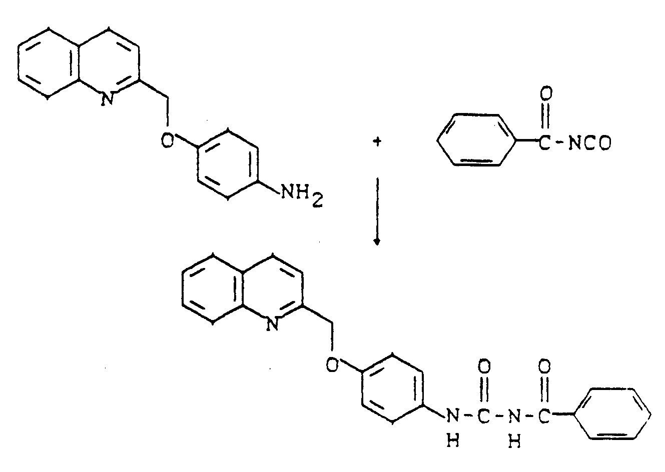
Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Das Verfahren wird im allgemeinen in einem Temperaturbereich von -80°C bis +80°C, bevorzugt von -80°C bis 0°C durchgeführt.

Das Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 3 Mol, bevorzugt 1 bis 2 Mol, besonders bevorzugt 1 Mol Isocyanat, bezogen auf 1 Mol des Amins, ein.

Die Aniline der allgemeinen Formel (II) sind an sich bekannt oder können nach üblicher Methode hergestellt werden [vgl. Ger. Offen. DE 36 07 382].

Die Isocyanate der allgemeinen Formel (III) sind bekannt oder können nach üblicher Methode hergestellt werden (vgl. Beilstein 9, S. 222).

Die erfindungsgemäßen substituierten (Chinolin-2-yl-methoxy)phenyl-carbonylharnstoffe können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen substituierten (Chinolin-2-yl-methoxy)phenyl-carbonylharnstoffe können sowohl in der Humanmedizin als auch in der Veterinärmedizin angewendet werden.

Die pharmakologischen Wirkdaten der erfindungsgemäßen Substanzen werden durch folgende Methode bestimmt:
Als Maß für die Lipoxygenase-Hemmung wurde die Freisetzung von Leukotrien B₄ (LTB₄) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148 - 2152 (1979), bestimmt. Die in vivo-Aktivität der Verbindungen wurde mit dem Mäuseohr-Entzündungsmodell nach Young, J.M. et al., J. of Investigative Dermatology 82, 367 - 371, (1984) nachgewiesen.

In der Tabelle 1 ist beispielhaft der nach diesem Test erzielte Wert einer erfindungsgemäßen Verbindung aufgeführt:

**Tabelle 1**

| Beispiel | LO-Hemmung IC₅₀ (mol/l) |
|---|---|
| 1 | 5,3 x 10⁻⁸ |

Die neuen Wirkstoffe können in an sich bekannter Weise unter Verwendung inerter, nichttoxischer, pharmazeutisch geeigneter Trägerstoffe oder Lösungsmittel in die üblichen Formulierungen überführt werden wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge aus zukommen, während in anderen Fällen die genannte obere Grenze überschritten werden muß. Im Fall der Applikation größerer Mengen kann es empfehlenswert sein, diese in mehreren Einzelgaben über den Tag zu verteilen.

### Ausgangsverbindung

### Beispiel I

### 2-(4-Aminophenoxymethyl)chinolin

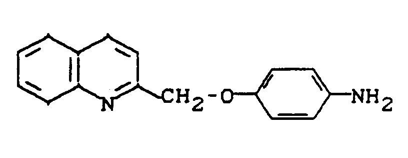

117,8 g (0,42 mol) 2-(4-Nitrophenoxymethyl)chinolin wurden in 1 l Methanol/Tetrahydrofuran (1:1) gelöst. Anschließend wurden ca. 5 g Raney-Nickel zugegeben und auf 35°C erwärmt. Dann wurden 63,1 g (1,26 mol) Hydrazinhydrat zugetropft und über Nacht gerührt. Es wurde vom Rückstand abfiltriert, die Lösung im Vakuum eingeengt und der Rückstand mit Methylenchlorid aufgenommen. Danach wurde mit Wasser gewaschen und die organische Phase mit konz. Salzsäure versetzt. Der ausgefallene Niederschlag wurde abfiltriert, mit 2 N Salzsäure gewaschen, in Wasser gelöst und mit 20%iger NaOH alkalisiert. Der Rückstand wurde im Vakuum getrocknet. Ausbeute: 88,0 g (92,1% der Theorie) Fp. = 131°C

### Herstellungsbeispiele

### Beispiel 1

### N-Benzoyl-N'-4-(chinolin-2-yl-methoxy)phenyl-harnstoff

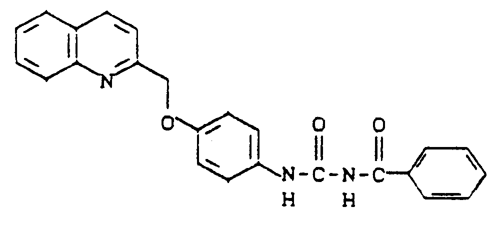

2,5 g (0,01 mol) der Verbindung aus Beispiel 1 werden in 100 ml Dichlormethan gelöst, unter Argonatmosphäre auf -80°C abgekühlt und tropfenweise mit 1,65 g (0,01 mol) Benzoylisocyanat (90%ig) (käuflich bei Aldrich) in 20 ml Dichlormethan versetzt. Beim Zusammengeben fällt sofort ein farbloser Niederschlag an. Man rührt noch 1 h bei -80°C nach, läßt die Temperatur dann auf Raumtemperatur ansteigen und filtriert den Niederschlag ab.
Ausbeute: 3,6 g (90,7% der Theorie) farblose Kristalle
Fp.: 220°C (Zers.)

### Beispiel 2

### N-Benzoyl-N′-3-(Chinolin-2-yl-methoxy)phenyl-harnstoff

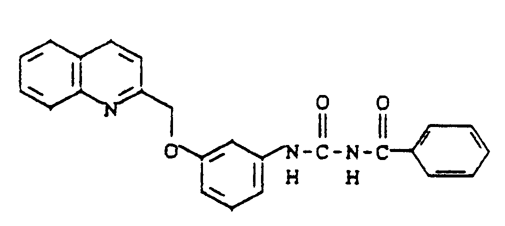

Die Titelverbindung wird in Analogie zu Beispiel 1 aus 2,5 g (0,01 mol) 2-(3-Amino-phenoxymethyl)-chinolin und 1,65 g (0,01 mol) Benzoylisocyanat (90%ig) hergestellt.
Ausbeute: 3,9 g (98,2% der Theorie) farblose Kristalle
Fp.: 180°C (Zers.)

### Beispiel 3

### N-2,6-Difluorbenzoyl-N'-3-(chinolin-2-yl-methoxy)-phenyl harnstoff

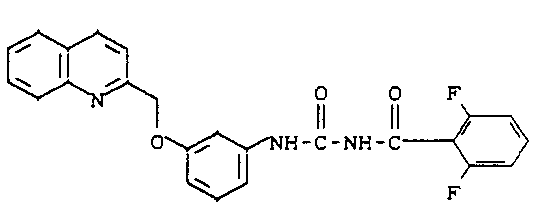

Die Titelverbindung wurde in Analogie zur Vorschrift der Beispiele 1 und 2 hergestellt.
Fp.: 180-182°C.

## Patentansprüche (Patentansprüche für folgende(n) Vertragsstaat(en): AT, BE, CH, DE, DK, FR, GB, GR, IT, LI, LU, NL, SE)

1. Substituierte (Chinolin-2-yl-methoxy)phenyl-carbonylharnstoffe der allgemeinen Formel 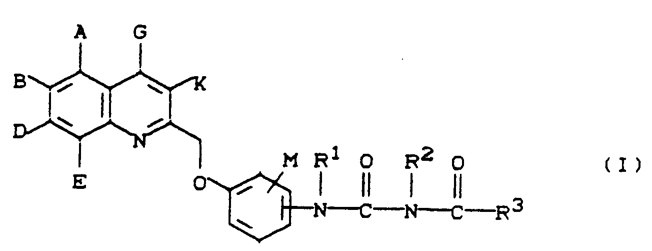 in welcher
A, B, D, E, G, K und M gleich oder verschieden sind und
- für Wasserstoff, Hydroxy, Halogen, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
R¹ und R² gleich oder verschieden sind und
- für Wasserstoff oder für geradkettiges oder verzweigtes Alkyl mit bis zu 10 Kohlenstoffatomen stehen, das gegebenenfalls durch Halogen, Aryl mit 6 bis 10 Kohlenstoffatomen oder durch Cycloalkyl mit 3 bis 8 Kohlenstoffatomen substituiert ist, oder
- für Cycloalkyl mit 3 bis 8 Kohlenstoffatomen stehen, oder
- für Aryl mit 6 bis 10 Kohlenstoffatomen stehen, das gegebenenfalls durch Halogen, Nitro, Cyano oder Trifluormethyl substituiert ist,
und
R³
- für Aryl mit 6 bis 10 Kohlenstoffatomen steht, das gegebenenfalls bis zu 3-fach gleich oder verschieden durch Halogen, Nitro, Cyano, Trifluormethyl oder durch geradkettiges oder verzweigtes Alkyl mit bis zu 8 Kohlenstoffatomen substituiert ist, oder
- für geradkettiges oder verzweigtes Alkyl mit bis zu 10 Kohlenstoffatomen steht, das gegebenenfalls durch Cycloalkyl mit 3 bis 8 Kohlenstoffatomen, Phenyl, Carboxy, Halogen, Hydroxy oder durch Alkoxy oder Alkoxycarbonyl mit jeweils bis zu 8 Kohlenstoffatomen substituiert ist, oder
- für Cycloalkyl mit 3 bis 8 Kohlenstoffatomen steht,
und deren Salze.

2. Substituierte (Chinolin-2-yl-methoxy)phenyl-carbonylharnstoffe der Formel (I) nach Anspruch 1
in welcher
A, B, D, E, G, K und M gleich oder verschieden sind und
- für Wasserstoff, Hydroxy, Fluor, Chlor, Brom, Carboxy, Nitro, Trifluormethyl, Trifluormethoxy oder für geradkettiges oder verzweigtes Alkyl oder Alkoxy mit jeweils bis zu 6 Kohlenstoffatomen stehen, oder
- für Phenyl stehen, das gegebenenfalls durch Fluor, Chlor, Brom, Hydroxy, Nitro oder Cyano substituiert ist,
R¹ und R² gleich oder verschieden sind und
- für Wasserstoff oder geradkettiges oder verzweigtes Alkyl mit bis zu 8 Kohlenstoffatomen stehen, das gegebenenfalls durch Fluor, Chlor, Brom, Phenyl, Cyclopropyl, Cyclopentyl oder Cyclohexyl substituiert ist, oder
- für Cyclopropyl, Cyclopentyl oder Cyclohexyl stehen, oder
- für Phenyl stehen, das gegebenenfalls durch Fluor, Chlor, Brom, Nitro, Cyano oder Trifluormethyl substituiert ist,
und
R³
- für Phenyl steht, das gegebenenfalls bis zu 2-fach gleich oder verschieden durch Fluor, Chlor, Brom, Nitro, Cyano, Trifluormethyl oder durch geradkettiges oder verzweigtes Alkyl mit bis zu 6 Kohlenstoffatomen substituiert ist, oder
- für geradkettiges oder verzweigtes Alkyl mit bis zu 8 Kohlenstoffatomen steht, das gegebenenfalls durch Cyclopropyl, Cyclopentyl oder Cyclohexyl, Fluor, Chlor, Brom, Hydroxy oder durch Alkoxy oder Alkoxycarbonyl mit jeweils bis zu 6 Kohlenstoffatomen substituiert ist, oder
- für Cyclopropyl, Cyclopentyl oder Cyclohexyl steht
und deren Salze.

3. Substituierte (Chinolin-2-yl-methoxy)phenyl-carbonylharnstoffe der Formel (I) nach Anspruch 1
in welcher
A, B, D, E, G, K und M gleich oder verschieden sind und
- für Wasserstoff, Hydroxy, Fluor, Chlor, Brom oder geradkettiges oder verzweigtes Alkyl mit bis zu 4 Kohlenstoffatomen stehen,
R¹ und R² gleich oder verschieden sind und
- für Wasserstoff oder geradkettiges oder verzweigtes Alkyl mit bis zu 6 Kohlenstoffatomen stehen, das gegebenenfalls durch Phenyl oder Cyclohexyl substituiert ist, oder
- Cyclopentyl oder Cyclohexyl stehen, oder
- für Wasserstoff oder geradkettiges oder verzweigtes Alkyl mit bis zu 6 Kohlenstoffatomen stehen, das gegebenenfalls durch Phenyl oder Cyclohexyl substituiert ist, oder
- für Cyclopentyl oder Cyclohexyl stehen, oder
R³
- für Phenyl steht, das gegebenenfalls bis zu 2-fach gleich oder verschieden durch Fluor, Chlor, Brom, Nitro oder Cyano substituiert ist, oder
- für geradkettiges oder verzweigtes Alkyl mit bis zu 6 Kohlenstoffatomen steht, das gegebenenfalls durch Cyclopropyl, Cyclopentyl oder Cyclohexyl substituiert ist, oder
- für Cyclopropyl, Cyclopentyl oder Cyclohexyl steht
und deren Salze.

4. Substituierte (Chinolin-2-yl-methoxy)phenyl-carbonylharnstoffe nach Anspruch 1 zur Bekämpfung von Krankheiten.

5. Verfahren zur Herstellung von substituierten (Chinolin-2-yl-methoxy)phenyl-carbonylharnstoffen der allgemeinen Formel 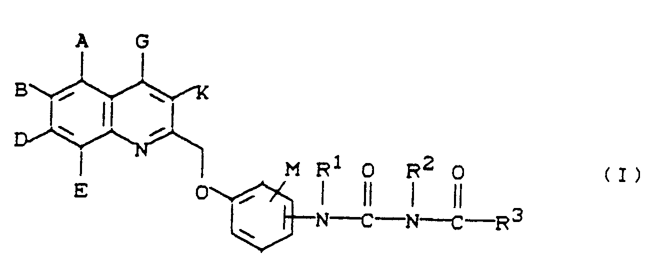 in welcher
A, B, D, E, G, K und M gleich oder verschieden sind und
- für Wasserstoff, Hydroxy, Halogen, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
R¹ und R² gleich oder verschieden sind und
- für Wasserstoff oder für geradkettiges oder verzweigtes Alkyl mit bis zu 10 Kohlenstoffatomen stehen, das gegebenenfalls durch Halogen, Aryl mit 6 bis 10 Kohlenstoffatomen oder durch Cycloalkyl mit 3 bis 8 Kohlenstoffatomen substituiert ist, oder
- für Cycloalkyl mit 3 bis 8 Kohlenstoffatomen stehen, oder
- für Aryl mit 6 bis 10 Kohlenstoffatomen stehen, das gegebenenfalls durch Halogen, Nitro, Cyano oder Trifluormethyl substituiert ist,
und
R³
- für Aryl mit 6 bis 10 Kohlenstoffatomen steht, das gegebenenfalls bis zu 3-fach gleich oder verschieden durch Halogen, Nitro, Cyano, Trifluormethyl oder durch geradkettiges oder verzweigtes Alkyl mit bis zu 8 Kohlenstoffatomen substituiert ist, oder
- für geradkettiges oder verzweigtes Alkyl mit bis zu 10 Kohlenstoffatomen steht, das gegebenenfalls durch Cycloalkyl mit 3 bis 8 Kohlenstoffatomen, Phenyl, Carboxy, Halogen, Hydroxy oder durch Alkoxy oder Alkoxycarbonyl mit jeweils bis zu 8 Kohlenstoffatomen substituiert ist, oder
- für Cycloalkyl mit 3 bis 8 Kohlenstoffatomen steht,
und deren Salze, dadurch gekennzeichnet, daß man Chinolylmethoxyaniline der allgemeinen Formel (II) 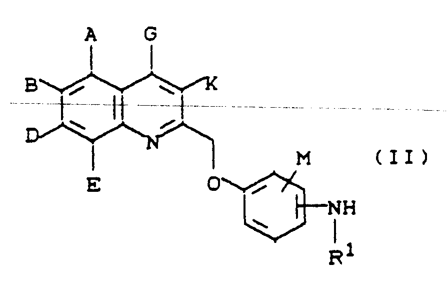 in welcher
A, B, D, E, G, K, M und R¹ die oben angegebene Bedeutung haben,
mit Isocyanaten der allgemeinen Formel (III)
R³-CO-NCO (III)
in welcher
R³ die oben angegebene Bedeutung hat,
in inerten Lösemitteln zu Verbindungen der allgemeinen Formel 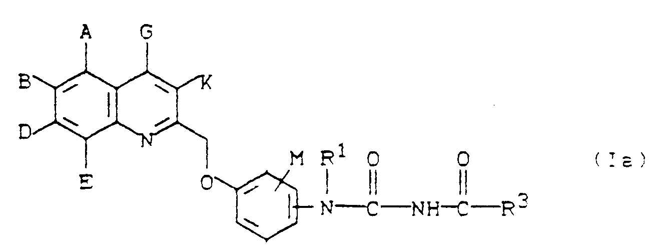 in welcher
A, B, D, E, G, K, M, R¹ und R³ die oben angegebene Bedeutung haben,
umsetzt und im Fall der Herstellung der Verbindungen der allgemeinen Formel (I) mit R² ≠ H anschließend die Verbindungen der Formel (Ia) mit Alkylierungsmitteln nach üblicher Methode in inerten Lösemitteln alkyliert.

6. Arzneimittel enthaltend mindestens einen substituierten (Chinolin-2-yl-methoxy)phenyl-carbonylharnstoff nach Anspruch 1.

7. Verfahren zur Herstellung von Arzneimitteln nach Anspruch 6, dadurch gekennzeichnet, das man Verbindungen nach Anspruch 1 gegebenenfalls mit Hilfe von üblichen Hilfs- und Trägerstoffen in eine geeignete Applikationsform bringt.

8. Verwendung der substituierten (Chinolin-2-yl-methoxy)phenyl-carbonylharnstoffe nach Anspruch 1 zur Herstellung von Arzneimitteln.

9. Verwendung der substituierten (Chinolin-2-yl-methoxy)phenyl-carbonylharnstoffe nach Anspruch 1 zur Herstellung von Arzneimitteln zur Hemmung enzymatischer Reaktionen im Rahmen des Arachidonsäuremetabolismus.

## Patentansprüche (Patentansprüche für folgende(n) Vertragsstaat(en): ES)

1. Verfahren zur Herstellung von substituierten (Chinolin-2-yl-methoxy)phenyl-carbonylharnstoffen der allgemeinen Formel 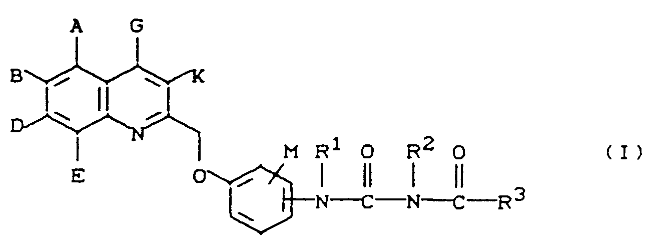 in welcher
A, B, D, E, G, K und M gleich oder verschieden sind und
- für Wasserstoff, Hydroxy, Halogen, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder
- für Aryl mit 6 bis 10 Kohlenstoffatomen stehen, das gegebenenfalls durch Halogen, Hydroxy, Nitro oder Cyano substituiert ist,
R¹ und R² gleich oder verschieden sind und
- für Wasserstoff oder für geradkettiges oder verzweigtes Alkyl mit bis zu 10 Kohlenstoffatomen stehen, das gegebenenfalls durch Halogen, Aryl mit 6 bis 10 Kohlenstoffatomen oder durch Cycloalkyl mit 3 bis 8 Kohlenstoffatomen substituiert ist, oder
- für Cycloalkyl mit 3 bis 8 Kohlenstoffatomen stehen, oder
- für Aryl mit 6 bis 10 Kohlenstoffatomen stehen, das gegebenenfalls durch Halogen, Nitro, Cyano oder Trifluormethyl substituiert ist,
und
R³
- für Aryl mit 6 bis 10 Kohlenstoffatomen steht, das gegebenenfalls bis zu 3-fach gleich oder verschieden durch Halogen, Nitro, Cyano, Trifluormethyl oder durch geradkettiges oder verzweigtes Alkyl mit bis zu 8 Kohlenstoffatomen substituiert ist, oder
- für geradkettiges oder verzweigtes Alkyl mit bis zu 10 Kohlenstoffatomen steht, das gegebenenfalls durch Cycloalkyl mit 3 bis 8 Kohlenstoffatomen, Phenyl, Carboxy, Halogen, Hydroxy oder durch Alkoxy oder Alkoxycarbonyl mit jeweils bis zu 8 Kohlenstoffatomen substituiert ist, oder
- für Cycloalkyl mit 3 bis 8 Kohlenstoffatomen steht,
und deren Salze, dadurch gekennzeichnet, daß man Chinolylmethoxyaniline der allgemeinen Formel (II) 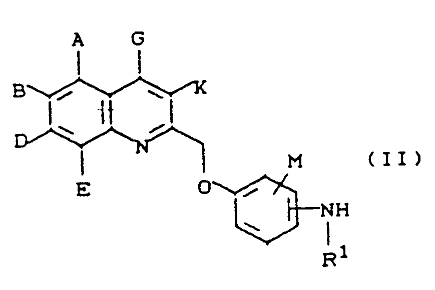 in welcher
A, B, D, E, G, K, M und R¹ die oben angegebene Bedeutung haben,
mit Isocyanaten der allgemeinen Formel (III)
R³-CO-NCO (III)
in welcher
R³ die oben angegebene Bedeutung hat,
in inerten Lösemitteln zu Verbindungen der allgemeinen Formel 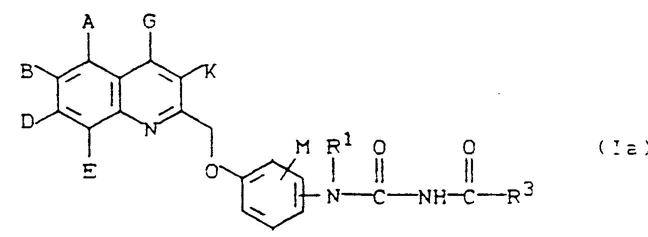 in welcher
A, B, D, E, G, K, M, R¹ und R³ die oben angegebene Bedeutung haben,
umsetzt und im Fall der Herstellung der Verbindungen der allgemeinen Formel (I) mit R² ≠ H anschließend die Verbindungen der Formel (Ia) mit Alkylierungsmitteln nach üblicher Methode in inerten Lösemitteln alkyliert.

## Claims (Claims for the following Contracting State(s): AT, BE, CH, DE, DK, FR, GB, GR, IT, LI, LU, NL, SE)

1. Substituted (quinolin-2-yl-methoxy)phenyl-carbonylureas of the general formula 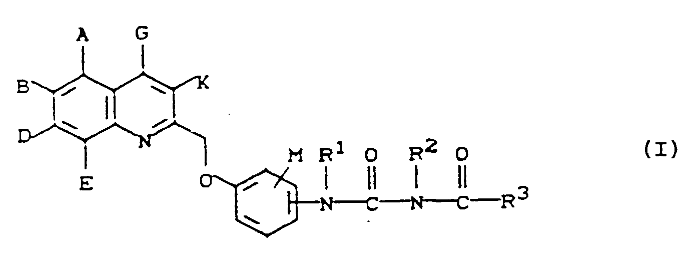 in which
A, B, D, E, G, K and M are identical or different and
- represent hydrogen, hydroxyl, halogen, carboxyl, nitro, trifluoromethyl, trifluoromethoxy or straight-chain or branched alkyl or alkoxy in each case having up to 8 carbon atoms, or
- represent aryl having 6 to 10 carbon atoms, which is optionally substituted by halogen, hydroxyl, nitro or cyano,
R¹ and R² are identical or different and
- represent hydrogen or straight-chain or branched alkyl having up to 10 carbon atoms, which is optionally substituted by halogen, aryl having 6 to 10 carbon atoms or by cycloalkyl having 3 to 8 carbon atoms, or
- represent cycloalkyl having 3 to 8 carbon atoms, or
- represent aryl having 6 to 10 carbon atoms, which is optionally substituted by halogen, nitro, cyano or trifluoromethyl,
and
R³
- represents aryl having 6 to 10 carbon atoms, which is optionally monosubstituted to trisubstituted by identical or different substituents from the series comprising halogen, nitro, cyano, trifluoromethyl or straight-chain or branched alkyl having up to 8 carbon atoms, or
- represents straight-chain or branched alkyl having up to 10 carbon atoms, which is optionally substituted by cycloalkyl having 3 to 8 carbon atoms, phenyl, carboxyl, halogen, hydroxyl or by alkoxy or alkoxycarbonyl in each case having up to 8 carbon atoms, or
- represents cycloalkyl having 3 to 8 carbon atoms,
and their salts.

2. Substituted (quinolin-2-yl-methoxy)phenyl-carbonylureas of the formula (I) according to Claim 1,
in which
A, B, D, E, G, K and M are identical or different and
- represent hydrogen, hydroxyl, fluorine, chlorine, bromine, carboxyl, nitro, trifluoromethyl, trifluoromethoxy or straight-chain or branched alkyl or alkoxy in each case having up to 6 carbon atoms, or
- represent phenyl which is optionally substituted by fluorine, chlorine, bromine, hydroxyl, nitro or cyano,
R¹ and R² are identical or different and
- represent hydrogen or straight-chain or branched alkyl having up to 8 carbon atoms, which is optionally substituted by fluorine, chlorine, bromine, phenyl, cyclopropyl, cyclopentyl or cyclohexyl, or
- represent cyclopropyl, cyclopentyl or cyclohexyl, or
- represent phenyl which is optionally substituted by fluorine, chlorine, bromine, nitro, cyano or trifluoromethyl,
and
R³
- represents phenyl which is optionally monosubstituted or disubstituted by identical or different substituents from the series comprising fluorine, chlorine, bromine, nitro, cyano, trifluoromethyl or straight-chain or branched alkyl having up to 6 carbon atoms, or
- represents straight-chain or branched alkyl having up to 8 carbon atoms, which is optionally substituted by cyclopropyl, cyclopentyl or cyclohexyl, fluorine, chlorine, bromine, hydroxyl or by alkoxy or alkoxycarbonyl in each case having up to 6 carbon atoms, or
- represents cyclopropyl, cyclopentyl or cyclohexyl
and their salts.

3. Substituted (quinolin-2-yl-methoxy)phenyl-carbonylureas of the formula (I) according to Claim 1,
in which
A, B, D, E, G, K and M are identical or different and
- represent hydrogen, hydroxyl, fluorine, chlorine, bromine or straight-chain or branched alkyl having up to 4 carbon atoms,
R¹ and R² are identical or different and
- represent hydrogen or straight-chain or branched alkyl having up to 6 carbon atoms, which is optionally substituted by phenyl or cyclohexyl, or
- represent cyclopentyl or cyclohexyl, or
- represent phenyl which is optionally substituted by fluorine, chlorine or bromine,
and
R³
- represents phenyl which is optionally monosubstituted or disubstituted by identical or different substituents from the series comprising fluorine, chlorine, bromine, nitro or cyano, or
- represents straight-chain or branched alkyl having up to 6 carbon atoms, which is optionally substituted by cyclopropyl, cyclopentyl or cyclohexyl, or
- represents cyclopropyl, cyclopentyl or cyclohexyl
and their salts.

4. Substituted (quinolin-2-yl-methoxy)phenyl-carbonylureas according to Claim 1 for combating diseases.

5. Process for the preparation of substituted (quinolin-2-yl-methoxy)phenyl-carbonylureas of the general formula 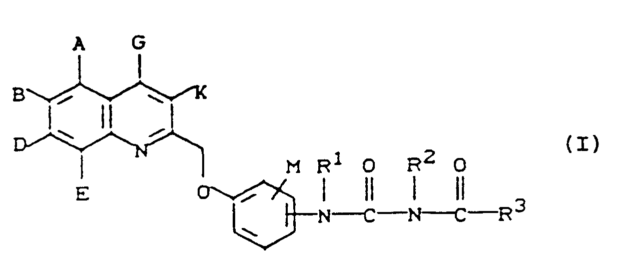 in which
A, B, D, E, G, K and M are identical or different and
- represent hydrogen, hydroxyl, halogen, carboxyl, nitro, trifluoromethyl, trifluoromethoxy or straight-chain or branched alkyl or alkoxy in each case having up to 8 carbon atoms, or
- represent aryl having 6 to 10 carbon atoms, which is optionally substituted by halogen, hydroxyl, nitro or cyano,
R¹ and R² are identical or different and
- represent hydrogen or straight-chain or branched alkyl having up to 10 carbon atoms, which is optionally substituted by halogen, aryl having 6 to 10 carbon atoms or by cycloalkyl having 3 to 8 carbon atoms, or
- represent cycloalkyl having 3 to 8 carbon atoms, or
- represent aryl having 6 to 10 carbon atoms, which is optionally substituted by halogen, nitro, cyano or trifluoromethyl,
and
R³
- represents aryl having 6 to 10 carbon atoms, which is optionally monosubstituted to trisubstituted by identical or different substituents from the series comprising halogen, nitro, cyano, trifluoromethyl or straight-chain or branched alkyl having up to 8 carbon atoms, or
- represents straight-chain or branched alkyl having up to 10 carbon atoms, which is optionally substituted by cycloalkyl having 3 to 8 carbon atoms, phenyl, carboxyl, halogen, hydroxyl or by alkoxy or alkoxycarbonyl in each case having up to 8 carbon atoms, or
- represents cycloalkyl having 3 to 8 carbon atoms,
and their salts, characterized in that quinolylmethoxyanilines of the general formula (II) 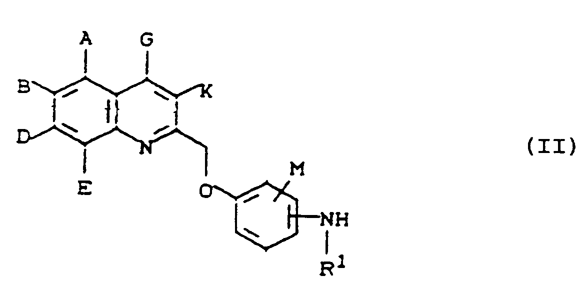 in which
A, B, D, E, G, K, M and R¹ have the abovementioned meaning,
are reacted with isocyanates of the general formula (III)
R³-CO-NCO (III)
in which
R³ has the abovementioned meaning,
in inert solvents to give compounds of the general formula 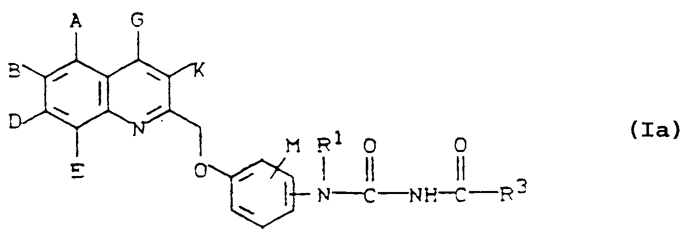 in which
A, B, D, E, G, K, M, R¹ and R³ have the above-mentioned meaning,
and in the case of the preparation of the compounds of the general formula (I) where R² ≠ H, the compounds of the general formula (Ia) are then alkylated in inert solvents with alkylating agents by a customary method.

6. Medicaments containing at least one substituted (quinolin-2-yl-methoxy)phenyl-carbonylurea according to Claim 1.

7. Method for the production of medicaments according to Claim 6, characterized in that compounds according to Claim 1 are brought into a suitable form for admini-stration, if appropriate with the aid of customary auxiliaries and excipients.

8. Use of the substituted (quinolin-2-yl-methoxy)phenyl-carbonylureas according to Claim 1 for the production of medicaments.

9. Use of the substituted (quinolin-2-yl-methoxy)phenyl-carbonylureas according to Claim 1 for the production of medicaments for the inhibition of enzymatic reactions in the context of arachidonic acid metabolism.

## Claims (Claims for the following Contracting State(s): ES)

1. Process for the preparation of substituted (quinolin-2-yl-methoxy)phenyl-carbonylureas of the general formula 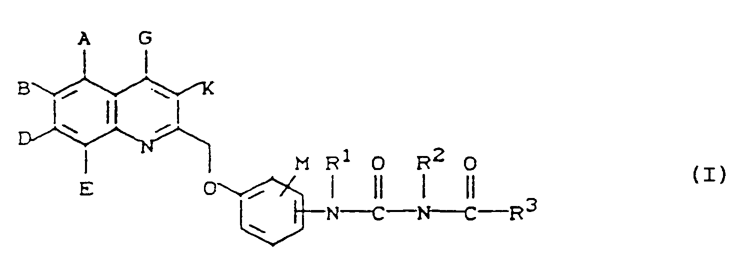 in which
A, B, D, E, G, K and M are identical or different and
- represent hydrogen, hydroxyl, halogen, carboxyl, nitro, trifluoromethyl, trifluoromethoxy or straight-chain or branched alkyl or alkoxy in each case having up to 8 carbon atoms, or
- represent aryl having 6 to 10 carbon atoms, which is optionally substituted by halogen, hydroxyl, nitro or cyano,
R¹ and R² are identical or different and
- represent hydrogen or straight-chain or branched alkyl having up to 10 carbon atoms, which is optionally substituted by halogen, aryl having 6 to 10 carbon atoms or by cycloalkyl having 3 to 8 carbon atoms, or
- represent cycloalkyl having 3 to 8 carbon atoms, or
- represent aryl having 6 to 10 carbon atoms, which is optionally substituted by halogen, nitro, cyano or trifluoromethyl,
and
R³
- represents aryl having 6 to 10 carbon atoms, which is optionally monosubstituted to trisubstituted by identical or different substituents from the series comprising halogen, nitro, cyano, trifluoromethyl or straight-chain or branched alkyl having up to 8 carbon atoms, or
- represents straight-chain or branched alkyl having up to 10 carbon atoms, which is optionally substituted by cycloalkyl having 3 to 8 carbon atoms, phenyl, carboxyl, halogen, hydroxyl or by alkoxy or alkoxycarbonyl in each case having up to 8 carbon atoms, or
- represents cycloalkyl having 3 to 8 carbon atoms,
and their salts, characterized in that quinolylmethoxyanilines of the general formula (II) 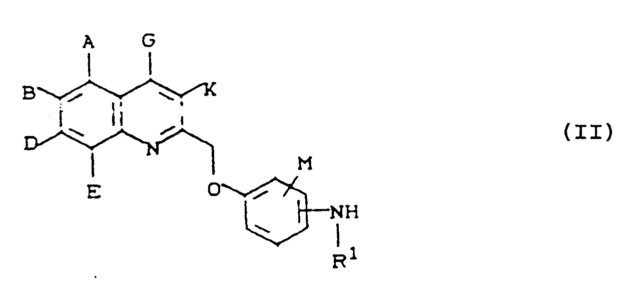 in which
A, B, D, E, G, K, M and R¹ have the abovementioned meaning,
are reacted with isocyanates of the general formula (III)
R³-CO-NCO (III)
in which
R³ has the abovementioned meaning,
in inert solvents to give compounds of the general formula 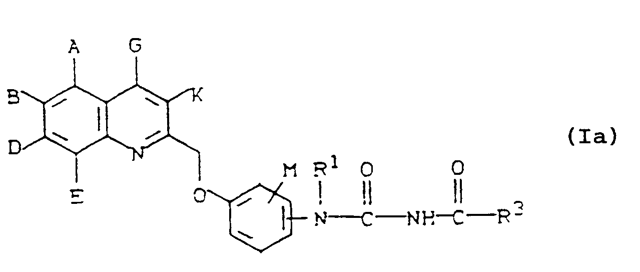 in which
A, B, D, E, G, K, M, R¹ and R³ have the above-mentioned meaning,
and in the case of the preparation of the compounds of the general formula (I) where R² ≠ H, the compounds of the general formula (Ia) are then alkylated in inert solvents with alkylating agents by a customary method.

## Revendications (Revendications pour l'(les) Etat(s) contractant(s) suivant(s): AT, BE, CH, DE, DK, FR, GB, GR, IT, LI, LU, NL, SE)

1. (Quinoléine-2-yl-méthoxy)-phénylcarbonylurées substituées de formule générale : 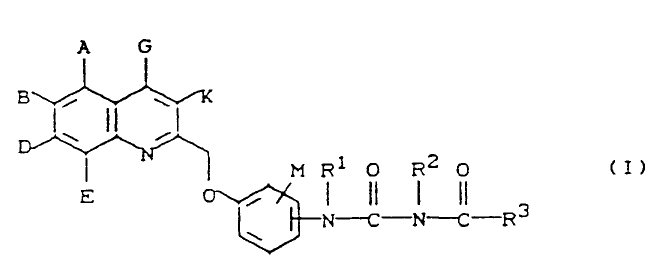 dans laquelle :
A, B, D, E, G, K et M, ayant des significations identiques ou différentes, représentent chacun
- l'hydrogène, un groupe hydroxy, un halogène, un groupe carboxy, nitro, trifluorométhyle, trifluorométhoxy ou un groupe alkyle ou alcoxy à chaîne droite ou ramifiée contenant chacun jusqu'à 8 atomes de carbone, ou bien
- un groupe aryle en C₆-C₁₀ éventuellement substitué par des halogènes, des groupes hydroxy, nitro ou cyano, R¹ et R², ayant des significations identiques ou différentes, représentent chacun
- l'hydrogène ou un groupe alkyle à chaîne droite ou ramifiée contenant jusqu'à 10 atomes de carbone et qui est éventuellement substitué par des halogènes, des groupes aryle en C₆-C₁₀ ou cycloalkyle en C₃-C₈, ou bien
- un groupe cycloalkyle en C₃-C₈, ou bien
- un groupe aryle en C₆-C₁₀ éventuellement substitué par des halogènes, des groupes nitro, cyano ou trifluorométhyle,
et
R³ représente
- un groupe aryle en C₆-C₁₀, portant éventuellement jusqu'à trois substituants identiques ou différents choisis parmi les halogènes, les groupes nitro, cyano, trifluorométhyle, ou un substituant alkyle à chaîne droite ou ramifiée contenant jusqu'à 8 atomes de carbone, ou bien
- un groupe alkyle à chaîne droite ou ramifiée contenant jusqu'à 10 atomes de carbone et éventuellement substitué par un groupe cycloalkyle en C₃-C₈, phényle, carboxy, des halogènes, des groupes hydroxy ou des groupes alcoxy ou alcoxycarbonyle contenant chacun jusqu'à 8 atomes de carbone, ou bien
- un groupe cycloalkyle en C₃-C₈,
et leurs sels.

2. (Quinoléine-2-yl-méthoxy)-phénylcarbonylurées substituées de formule I, selon la revendication 1,
dans laquelle
A, B, D, E, G, K et M, ayant des significations identiques ou différentes, représentent chacun
- l'hydrogène, un groupe hydroxy, le fluor, le chlore, le brome, un groupe carboxy, nitro, trifluorométhyle, trifluorométhoxy ou un groupe alkyle ou alcoxy à chaîne droite ou ramifiée contenant chacun jusqu'à 6 atomes de carbone, ou bien
- un groupe phényle éventuellement substitué par le fluor, le chlore, le brome, des groupes hydroxy, nitro ou cyano,
R¹ et R², ayant des significations identiques ou différentes, représentent chacun
- l'hydrogène ou un groupe alkyle à chaîne droite ou ramifiée contenant jusqu'à 8 atomes de carbone et qui est éventuellement substitué par le fluor, le chlore, le brome, des groupes phényle, cyclopropyle, cyclopentyle ou cyclohexyle, ou bien
- un groupe cyclopropyle, cyclopentyle ou cyclohexyle, ou bien
- un groupe phényle éventuellement substitué par le fluor, le chlore, le brome, des groupes nitro, cyano ou trifluorométhyle,
et
R³ représente
- un groupe phényle portant éventuellement jusqu'à deux substituants identiques ou différents choisis parmi le fluor, le chlore, le brome, les groupes nitro, cyano, trifluorométhyle, ou un substituant alkyle à chaîne droite ou ramifiée contenant jusqu'à 6 atomes de carbone, ou bien
- un groupe alkyle à chaîne droite ou ramifiée contenant jusqu'à 8 atomes de carbone et éventuellement substitué par des groupes cyclopropyle, cyclopentyle ou cyclohexyle, le fluor, le chlore, le brome, des groupes hydroxy ou des groupes alcoxy ou alcoxycarbonyle contenant chacun jusqu'à 6 atomes de carbone,
- un groupe cyclopropyle, cyclopentyle ou cyclohexyle, et leurs sels.

3. (Quinoléine-2-yl-méthoxy)-phénylcarbonylurées substituées de formule I selon la revendication 1,
dans laquelle
A, B, D, E, G, K et M, ayant des significations identiques ou différentes, représentent chacun
- l'hydrogène, un groupe hydroxy, le fluor, le chlore, le brome ou un groupe alkyle à chaîne droite ou ramifiée contenant jusqu'à 4 atomes de carbone, R¹ et R², ayant des significations identiques ou différentes, représentent chacun
- l'hydrogène ou un groupe alkyle à chaîne droite ou ramifiée contenant jusqu'à 6 atomes de carbone et qui est éventuellement substitué par un groupe phényle ou cyclohexyle, ou bien
- un groupe cyclopentyle ou cyclohexyle, ou bien
- l'hydrogène ou un groupe alkyle à chaîne droite ou ramifiée contenant jusqu'à 6 atomes de carbone et éventuellement substitué par un groupe phényle ou cyclohexyle, ou bien
- un groupe cyclopentyle ou cyclohexyle, ou bien
R³ représente
- un groupe phényle portant éventuellement jusqu'à deux substituants identiques ou différents choisis parmi le fluor, le chlore, le brome, les groupes nitro ou cyano, ou bien
- un groupe alkyle à chaîne droite ou ramifiée contenant jusqu'à 6 atomes de carbone et éventuellement substitué par un groupe cyclopropyle, cyclopentyle ou cyclohexyle, ou bien
- un groupe cyclopropyle, cyclopentyle ou cyclohexyle, et leurs sels.

4. (Quinoléine-2-yl-méthoxy)-phénylcarbonylurées substituées de formule I selon la revendication 1 pour le traitement de maladies.

5. Procédé de préparation des (quinoléine-2-yl-méthoxy)-phénylcarbonylurées substituées de formule générale : 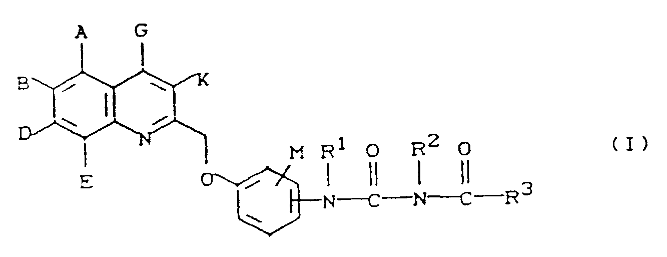 dans laquelle
A, B, D, E, G, K et M, ayant des significations identiques ou différentes, représentent chacun
- l'hydrogène, un groupe hydroxy, un halogène, un groupe carboxy, nitro, trifluorométhyle, trifluorométhoxy ou un groupe alkyle ou alcoxy à chaîne droite ou ramifiée contenant chacun jusqu'à 8 atomes de carbone, ou bien
- un groupe aryle en C₆-C₁₀ éventuellement substitué par des halogènes, des groupes hydroxy, nitro ou cyano, R¹ et R², ayant des significations identiques ou différentes, représentent chacun
- l'hydrogène ou un groupe alkyle à chaîne droite ou ramifiée contenant jusqu'à 10 atomes de carbone et qui est éventuellement substitué par des halogènes, des groupes aryle en C₆-C₁₀ ou des groupes cycloalkyle en C₃-C₈, ou bien
- un groupe cycloalkyle en C₃-C₈, ou bien
- un groupe aryle en C₆-C₁₀ éventuellement substitué par des halogènes, des groupes nitro, cyano ou trifluorométhyle,
et
R³ représente
- un groupe aryle en C₆-C₁₀ portant éventuellement jusqu'à trois substituants identiques ou différents choisis parmi les halogènes, les groupes nitro, cyano, trifluorométhyle ou un substituant alkyle à chaîne droite ou ramifiée contenant jusqu'à 8 atomes de carbone, ou bien
- un groupe alkyle à chaîne droite ou ramifiée contenant jusqu'à 10 atomes de carbone, éventuellement substitué par des groupes cycloalkyle en C₃-C₈, phényle, carboxy, des halogènes, des groupes hydroxy ou des groupes alcoxy ou alcoxycarbonyle contenant chacun jusqu'à 8 atomes de carbone, ou bien
- un groupe cycloalkyle en C₃-C₈,
et de leurs sels, caractérisé en ce que l'on fait réagir des quinoléylméthoxyanilines de formule générale II : 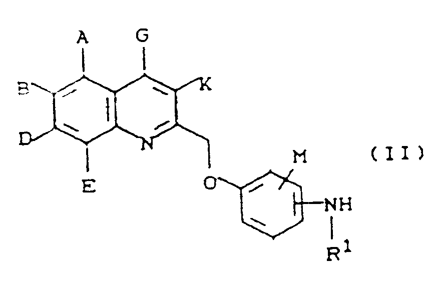 dans laquelle A, B, D, E, G, K et M ont les significations indiquées ci-dessus, avec des isocyanates de formule générale III :
R³-CO-NCO (III)
dans laquelle R³ a les significations indiquées ci-dessus,
dans des solvants inertes, la réaction donnant des composés de formule générale : 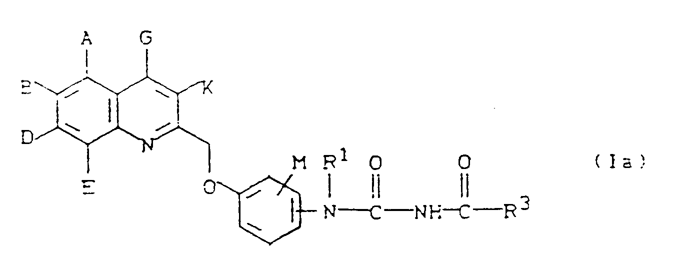 dans laquelle A, B, D, E, G, K, M, R¹ et R³ ont les significations indiquées ci-dessus,
et, dans le cas où l'on prépare des composés de formule générale I, dans laquelle R² a une signification autre que l'hydrogène, on alkyle ensuite les composés de formule Ia à l'aide d'agents alkylants par des techniques usuelles dans des solvants inertes.

6. Médicament contenant au moins une (quinoléine-2-yl-méthoxy)-phénylcarbonylurée substituée selon la revendication 1.

7. Procédé de préparation de médicaments selon la revendication 6, caractérisé en ce que l'on met des composés selon la revendication 1 sous une forme d'administration appropriée, éventuellement à l'aide de produits auxiliaires et véhicules usuels.

8. Utilisation des (quinoléine-2-yl-méthoxy)-phénylcarbonylurées substituées selon la revendication 1 pour la préparation des médicaments.

9. Utilisation des (quinoléine-2-yl-méthoxy)-phénylcarbonylurées substituées selon la revendication 1 pour la préparation de médicaments prévus pour inhiber les réactions enzymatiques dans le cadre du métabolisme de l'acide arachidonique.

## Revendications (Revendications pour l'(les) Etat(s) contractant(s) suivant(s): ES)

1. Procédé de préparation des (quinoléine-2-yl-méthoxy)-phénylcarbonylurées substituées de formule générale : 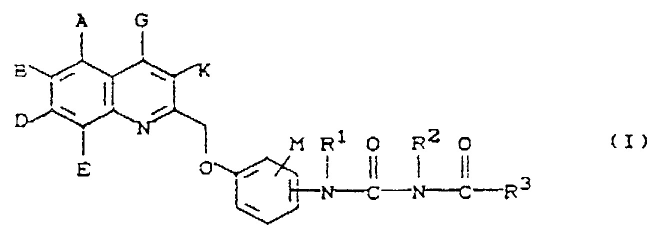 dans laquelle
A, B, E, D, G, K et M, ayant des significations identiques ou différentes, repré-sentent chacun
- l'hydrogène, un groupe hydroxy, un halogène, un groupe carboxy, nitro, trifluorométhyle, trifluorométhoxy ou un groupe alkyle ou alcoxy à chaîne droite ou ramifiée contenant chacun jusqu'à 8 atomes de carbone, ou bien
- un groupe aryle en C₆-C₁₀ éventuellement substitué par des halogènes, des groupes hydroxy, nitro ou cyano, R¹ et R², ayant des significations identiques ou différentes, représentent chacun
- l'hydrogène ou un groupe alkyle à chaîne droite ou ramifiée contenant jusqu'à 10 atomes de carbone et éventuellement substitué par des halogènes, des groupes aryle en C₆-C₁₀ ou cycloalkyle en C₃-C₈, ou bien
- un groupe cycloalkyle en C₃-C₈, ou bien
- un groupe aryle en C₆-C₁₀ éventuellement substitué par des halogènes, des groupes nitro, cyano ou trifluorométhyle,
et
R³ représente
- un groupe aryle en C₆-C₁₀ portant éventuellement jusqu'à trois substituants identiques ou différents choisis parmi les halogènes, les groupes nitro, cyano, trifluorométhyle ou un substituant alkyle à chaîne droite ou ramifiée contenant jusqu'à 8 atomes de carbone, ou bien
- un groupe alkyle à chaîne droite ou ramifiée contenant jusqu'à 10 atomes de carbone et éventuellement substitué par un groupe cycloalkyle en C₃-C₈, phényle, carboxy, des halogènes, des groupes hydroxy ou des groupes alcoxy ou alcoxycarbonyle contenant chacun jusqu'à 8 atomes de carbone, ou bien
- un groupe cycloalkyle en C₃-C₈,
et de leurs sels, caractérisé en ce que l'on fait réagir des quinoléylméthoxyanilines de formule générale : 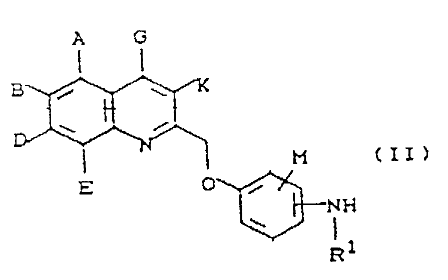 dans laquelle A, B, D, E, G, K, M et R¹ ont les significations indiquées ci-dessus, avec des isocyanates de formule générale III :
R³-CO-NCO (III)
dans laquelle R³ a les significations indiquées ci-dessus,
dans des solvants inertes, la réaction donnant des composés de formule générale : 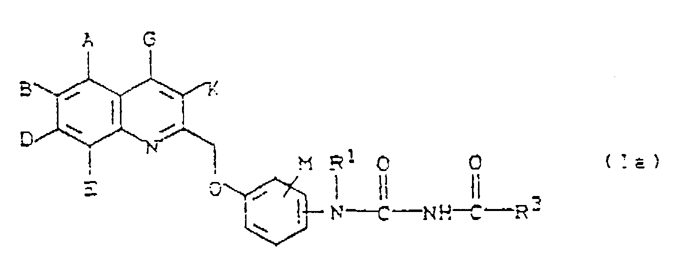 dans laquelle A, B, D, E, G, K, M, R¹ et R³ ont les significations indiquées ci-dessus,
et, dans le cas où l'on prépare des composés de formule générale I, pour lesquels R² a une signification autre que l'hydrogène, on alkyle ensuite les composés de formule Ia à l'aide d'agents alkylants par des modes opératoires usuels dans des solvants inertes.
